# EUROPEAN PATENT APPLICATION

(11) **EP 4 129 274 A1**
(43) Date of publication of application: **08.02.2023**
(21) Application number: 20927090.9
(22) Date of filing: 24.03.2020
(51) Int. Cl.: A61K 31/045, A61P 25/28, A61P 25/00, A61P 25/16, A61P 9/10

(54) **USE OF MENTHOL FOR PREPARING EXTERNAL COMPOSITION FOR TREATING NEURODEGENERATIVE DISEASE AND STROKE**

(71) Applicant: China Medical University, Taichung City 404 (TW)
(72) Inventor: CHEN, Yi-Hung, Taipei City, Taiwan 106 (TW); HUANG, Shiang-Suo, Taichung City, Taiwan 408 (TW); HUNG, Shih-Ya, Taichung City, Taiwan 406 (TW); SU, Hsing-Hui, Taichung City, Taiwan 402 (TW); WANG, Yi-Hsin, Tainan City, Taiwan 710 (TW); CHUNG, Hsin-Yi, Nantou County, Taiwan 545 (TW); LUO, Sih-Ting, Taichung City, Taiwan 426 (TW); CHEN, Chao-Jung, Taichung City, Taiwan 412 (CN); CHU, Yu-Ting, Changhua City, Changhua County, Taiwan 500 (TW); MACDONALD, Iona, Jean, Taichung City, Taiwan 404 (CN)
(74) Representative: Cabinet Chaillot
(86) International application number: PCT/CN2020/080917
(87) International publication number: WO 2021/189256

(57) **Abstract**

The use of menthol or an isomer thereof for preparation of a topical composition to improve neurodegenerative diseases and stroke wherein the neurodegenerative diseases are attributed to cerebral neurons impaired or degenerated, shortage of dopamine in a brain. The topical composition is manufactured as patches, liquids, pastes, oily substances, powders, gels, sprays, composite products or other products to be covered on limbs and applied on skin. A product to be covered on limbs can be a glove, a foot muff, a sock or an extended part or a layered object from a garment for continuous contact between skin and menthol. The present invention also provides the use of menthol or an isomer thereof for preparation of a topical composition to improve diseases or symptoms attributed to cerebral neurons impaired or degenerated, shortage of dopamine or stroke.

## Description

### BACKGROUND OF THE INVENTION

### 1. Field of the invention

The present invention relates to improvements of neurodegenerative diseases as well as stroke and provides and explores the use of menthol for a topical composition.

### 2. Description of the prior art

The neurodegenerative diseases such as Parkinson's disease and Alzheimer's disease have been a global issue and their pathogenesis is cerebral or spinal neurons continuously degenerating or dying with time that cause ataxia or mental defect (dementia). Currently, there is still no effective therapy to cure or prevent neurodegenerative diseases. In clinical detections, Parkinson's disease, i.e., a typical neurodegenerative disease also known as paralysis agitans, is characteristic of the level of dopamine in a patient's brain lower than the normal level and degeneration of two types of nucleus pigmentosus pontis (substantia nigra and locus coeruleus) in the brainstem. It is generally acknowledged that Parkinson's disease is attributed to degeneration of the pathway for dopaminergic projection from the compact part of substantia nigra to the corpus striatum because of a positive correlation between degenerative substantia nigra and decrease of dopamine in the striatum. The symptoms of Parkinson's disease are (1) resting tremor, (2) muscular rigidity, (3) difficulty in initiating voluntary actions and (4) bradykinesia. Parkinson's disease is still an incurable disease but moderated by administration of medications. However, medications are accompanied with side effects, for example, dyskinesia, and a high risk such as mental disease is derived from a high dose for worsened illness.

Strokes caused by the supply of cerebral blood flows blocked or abating are divided into three types clinically: ischemic stroke, hemorrhagic stroke and transient ischemic attack. For the incidence of stroke, more than 85% cases are classified as ischemic stroke. In a case of apoplectic stroke, brain cells are necrotic and accompanied with the fast partial or whole cerebral dysfunction clinically and the stroke patient die after 24 hours because of oxygen and nutrients not supplied to the brain tissues adequately. The stroke onset inducing nerve inflammations in brain tissues further leads to the brain injury. In the clinical treatment, medications are administrated to a patient for moderation of nerve inflammations and remission or prevention of stroke-induced complications usually. Currently, one typical medication to treat strokes is tissue-type plasminogen activator (tPA) which is administrated to a patient through the intravenous injection according to a careful assessment mechanism for any possible high risk of intracranial hemorrhage.

The patent related to the field of invention is described as follows:
The patent "Methods and compositions for the treatment of neurodegenerative disorders" (patent publication number: TW 200848063) introduces treatment of neurodegenerative disorders by oral blockers with which the passage of ions through the ion channel is blocked. Despite the efficiency from the experiment based on a cell line for the ion channel, any improvement for indications is not assessed in the patent and needs to be researched clinically.

In summary, how to provide an effective and simple method for improvement of neurodegenerative diseases and stroke without side effects is the topic explored in the present disclosure.

### SUMMARY OF THE INVENTION

The present invention provides the use of menthol or an isomer thereof for preparation of a topical composition to improve neurodegenerative diseases or stroke.

The isomer of menthol is a structural isomer or a stereoisomer; the menthol or the isomer thereof features a weight percentage ranging from 1% to 20%.

The topical composition is manufactured as patches, liquids, pastes, oily substances, powders, gels, sprays, composite products or other products to be covered on limbs and applied on skin. A product to be covered on limbs can be a glove, a foot muff, a sock or an extended part or a layered object from a garment for continuous contact between menthol and skin.

The neurodegenerative diseases such as Parkinson's disease and Alzheimer's disease are attributed to cerebral neurons impaired or degenerated or shortage of dopamine in a brain. The stroke means ischemic stroke; the topical composition is used in improving symptoms attributed to cerebral neurons impaired or degenerated, shortage of dopamine or stroke and including, without limitation, cognitive dysfunction, ataxia, depression, weakness of limbs, hemiplegia, headache, vertigo, aphasia or alalia, blurring of vision or visual impairment, palpitation, convulsion, nausea and vomiting, fatigue, drooling, dysphagia, facial paralysis and incontinence.

The present invention provides the use of menthol or an isomer thereof for preparation of a topical composition to improve diseases or symptoms attributed to cerebral neurons impaired or degenerated, shortage of dopamine or stroke.

In the present disclosure, it is demonstrated in one embodiment for the Parkinson's disease mouse model that the impaired sports coordination due to Parkinson's disease and the level of dopamine in a mouse's brain are improved with topical menthol and the topical menthol with the effect of neuroprotection prevents dopamine neurons in substantia nigra from impairments and raises the level of TH. As shown in another embodiment for the ischemic stroke mouse model, the cerebral infarction volume decreases within seven days for correction of the defect in sensorimotor nerves with topical menthol applied on mice.

In the present disclosure, the stereotypes that a neurodegenerative disease or a stroke cannot be cured and administrations of medicines are accompanied with serious side effects are overturned and the efficiencies are shown as follows:

### (1) Topical composition easy to use

A topical therapy first introduced in the present disclosure, which is different from conventional medicine therapies applicable through unnerving injection or oral administration referring to a regular schedule or inconvenient condition assessment for neurodegenerative diseases or strokes, is an effective therapeutic method through application easy and unrestricted and menthol applied, sprayed, soaked or coated on skin.

### (2) No side effect

The topical menthol dissimilar to existing medications for neurodegenerative diseases or strokes that are criticized for risks of side effects, dosage and mixture with another medicine to subdue side effects is a safe and side-effect-free substance with which another therapy or medical formula incorporates flexibly.

### (3) Dual effects for improvement of neurodegenerative diseases and stroke

The new application of menthol in the present disclosure, which is verified for better neuroprotection, increasing cerebral dopamine and subdued cerebral infarction by rigorous and complete animal experiments, is available for pathogeneses of neurodegenerative diseases and stroke through topical administration only, proves effective in improvement that obliges patients and protects them from discomforts or side effects due to administrations of medicines prescribed, and promotes patients' quality of life.

### (4) Extensive application for improvement of diseases

With functions recognized such as neuroprotection, increasing cerebral dopamine and subdued cerebral infarction, menthol is expected to improve other diseases or symptoms with similar pathogeneses in addition to neurodegenerative diseases and stroke and critically acclaimed for low side effects and topical administration contributing to wide marketing development potential and industrial application value.

### BRIEF DESCRIPTION OF THE DRAWINGS

FIG. 1A illustrates an experiment design in **Embodiment 1.**
FIG. 1B illustrates the retention time for Parkinson's disease mice on a rotating rod.
FIG. 1C illustrates the level of dopamine in the corpus striatum for Parkinson's disease mice in each group.
FIG. 1D illustrates tyrosine hydroxylase (TH) presented in substantia nigra for Parkinson's disease mice in each group.
FIG. 2A illustrates an experiment design in **Embodiment 2.**
FIG. 2B-1 and FIG. 2B-2 illustrate the retention time for ischemic stroke mice on a rotating rod, respectively.
FIG. 2C-1 illustrates the time for stickers found by ischemic stroke mice in each group.

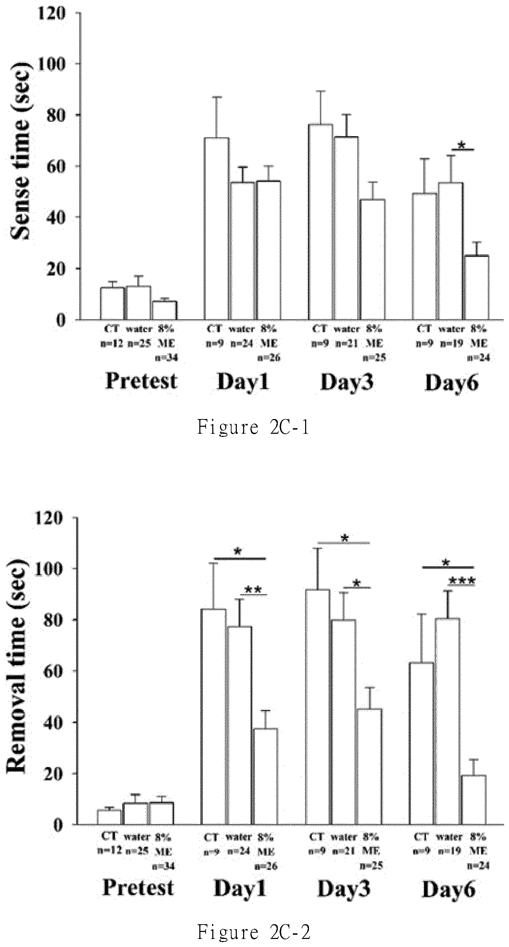

FIG. 2C-2 illustrates the time for stickers removed by ischemic stroke mice in each group.

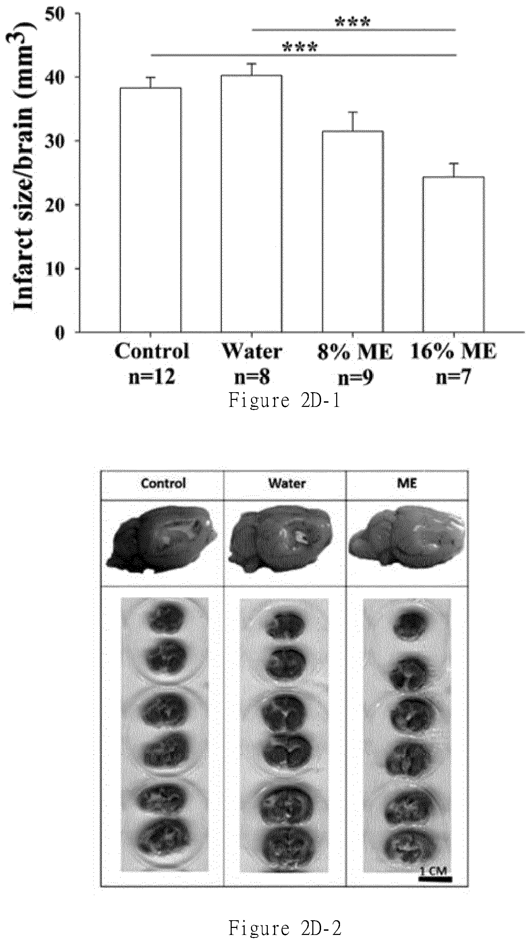

FIG. 2D-1 illustrates the measured cerebral infarction volume for ischemic stroke mice in each group.
FIG. 2D-2 illustrates brain specimens collected from ischemic stroke mice in each group and stained with TTC (2,3,5-triphenyltetrazolium chloride).

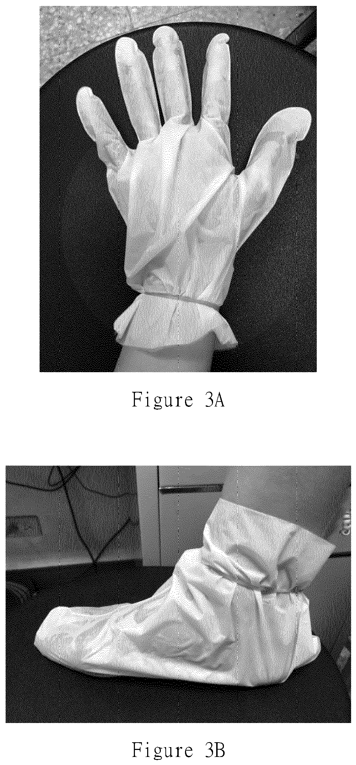

FIG. 3A menthol illustrates a glove, that is, a product embodied with the topical composition.
FIG. 3B menthol illustrates a foot muff, that is, another product embodied with the topical composition.

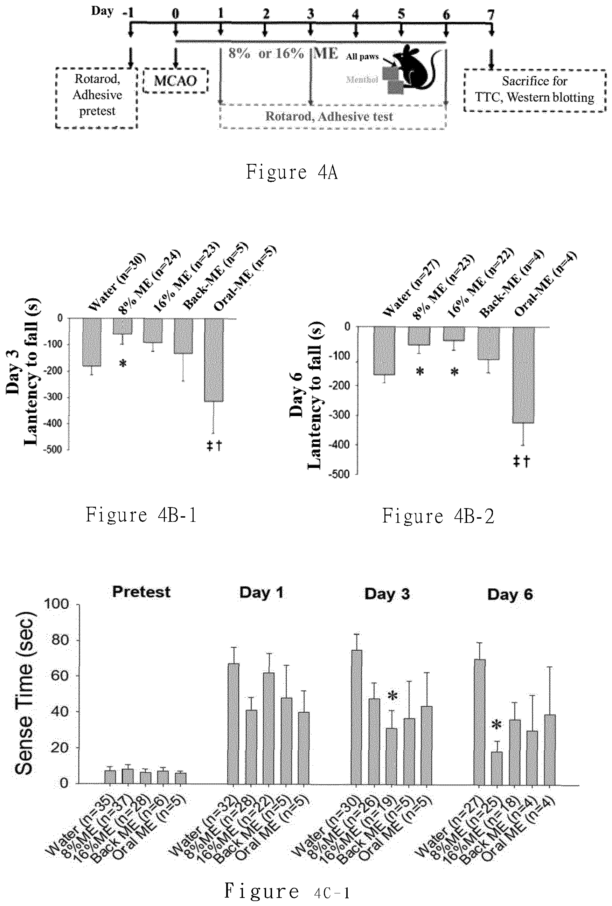

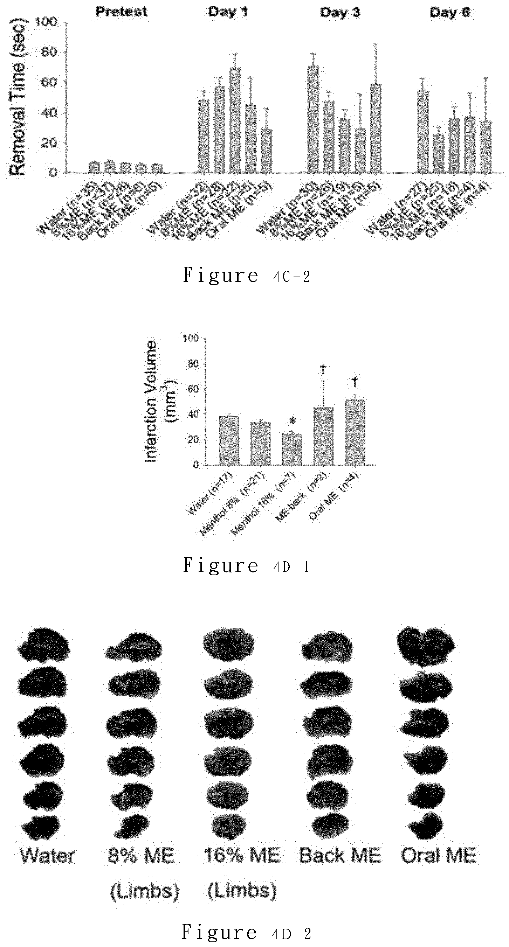

FIG. 4A illustrates an experiment design for ischemic stroke mice which are fed with menthol or whose limbs or backs are soaked in menthol.
FIG. 4B-1 and FIG. 4B-2 illustrate the retention time for ischemic stroke mice on a rotating rod, respectively.
FIG. 4C-1 and FIG. 4C-2 illustrate the time for stickers found by ischemic stroke mice in each group.
FIG. 4D-1 and FIG. 4D-2 illustrate the measured cerebral infarction volume for ischemic stroke mice in each group, respectively.

### DETAILED DESCRIPTION OF THE PREFERRED EMBODIMENT

The technical and scientific terminologies in the patent specification are commonly understood by persons skilled in the art unless otherwise specified. In the present disclosure, the terminology of "MPTP" means 1-methyl-4-phenyl-1,2,3,6-tetrahydropyridine, that is, a neurotoxin to cause the symptom of Parkinson's disease by destroying neurons that generate dopamine in substantia nigra (SN), and is injected into a mouse for creation of the Parkinson's disease mouse model. The terminology of "LC-MS/MS" means liquid chromatography-tandem mass spectrometry. The terminology of "TH" means tyrosine hydroxylase which is an enzyme necessary to generation of dopamine from cells. The terminology of "substantia nigra (SN)" means nerve nuclei at mesencephalon which contain cells with TH generating dopamine and being critical to the symptom of Parkinson's disease after apoptosis. The terminology of "MCAO" means middle cerebral artery occlusion which is a technique for creation of an animal model of ischemic stroke. The terminology of "TTC" means 2,3,5-triphenyltetrazolium chloride, that is, a redox indicator which is reduced to TPF (1,3,5-triphenylformazan; a red compound) by dehydrogenase (particularly succinodehydrogenase in mitochondria) in living cells but not reduced by inactive dehydrogenase in necrotic tissues displaying pale in contrast to crimson normal tissues; TTC is taken as a staining agent with which an ischemic infarct tissue can be detected.

Some specific embodiments are shown in following sections for further elaborating technical features and effects of the present invention.

### Embodiment 1: topical menthol for improvement of Parkinson's disease

### 1. Experimental design and creation of a Parkinson's disease mouse model

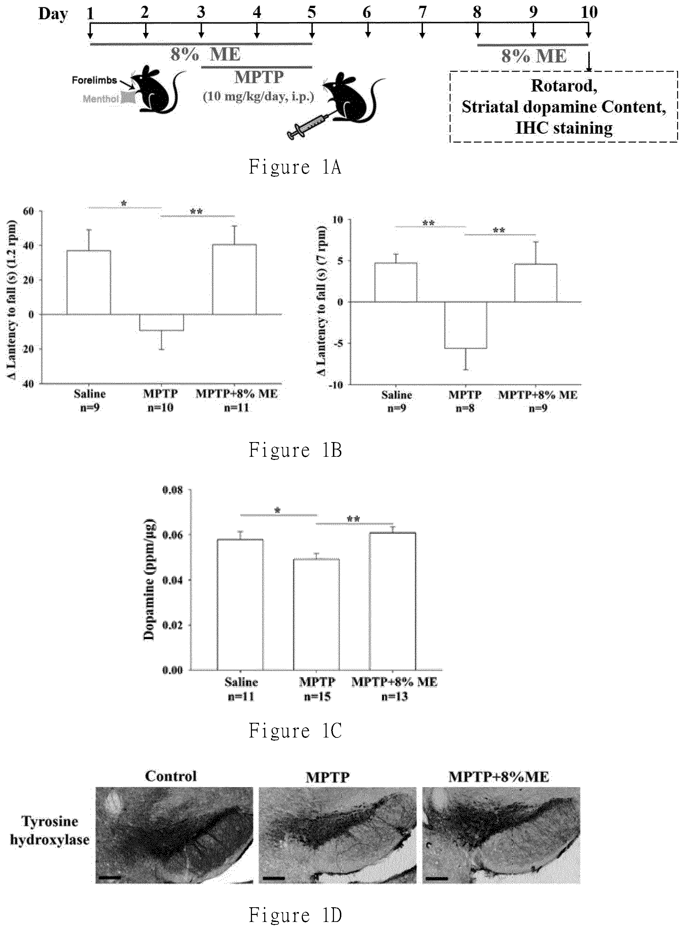

Laboratory mice are divided into three groups: "Control" consisting of normal mice; "MPTP" consisting of Parkinson's disease mice fed with MPTP for creation of a disease model; "MPTP+8%ME" consisting of Parkinson's disease mice which are fed with MPTP for creation of a disease model and whose forepaws are soaked in 8% menthol. The experiment schedule is indicated in FIG. 1A. For administration of menthol, two forepaws of each "MPTP+8%ME" mouse are soaked in 8% (wt%) menthol solution for 10 seconds per day from Day 1 to Day 5 and from Day 8 to Day 10, respectively. For creation of a Parkinson's disease mouse model, MPTP (daily dosage: 10mg/kg) is injected into "MPTP" and "MPTP+8%ME" mice through the intraperitoneal (I.P.) injection on three consecutive days from Day 3 to Day 5. By Day 10, the endurance exercise test, the dopamine assay and the immunohistochemical staining are conducted for any improvement in the symptom of Parkinson's disease due to menthol.

### 2. Endurance exercise test - Rotarod test

The rotarod test is a performance test in which a rodent keeps running on a self-accelerated rotating rod activated from 4rpm (initial rotation rate) to 40 rpm (maximum and constant rotation rate in five minutes) on Day 1 (Day 10) for estimation of the retention time of the rodent on the rotating rod (FIG. 1B). As shown in the ordinate of FIG. 1B, "Δ latency to fall" denotes the difference between the retention time on Day 10 and the retention time on Day 1. It can be seen from test results that the retention time of MPTP mice on Day 10 significantly shorter than the retention time on Day 1 suggests worse sports coordination of Parkinson's disease mice. Comparatively, the retention time of MPTP+8%ME mice on Day 10 longer than the retention time on Day 1 suggests that impaired sports coordination of Parkinson's disease mice whose forepaws were soaked in menthol improves and these mice even performs as normal mice (Control group).

### 3. Dopamine assay

In the experiment to test the level of dopamine in a mouse's brain, the brain of a mouse sacrificed after the rotarod test is taken out for testing the level of dopamine in the corpus striatum by LC-MS/MS (FIG. 1C). As shown in test results, the level of dopamine in MPTP mice decreasing significantly on Day 10 suggests the typical symptom of Parkinson's disease. Comparatively, the level of dopamine in MPTP+8%ME mice similar to that of Control mice suggests the level of dopamine in Parkinson's disease mice is recovered.

### 4. Immunohistochemical (IHC) staining

The level of TH in a mouse's substantia nigra is tested through IHC staining (FIG. 1D). As shown in test results, the level of TH in MPTP+8%ME mice is recovered and similar to that of Control mice compared with the level of TH decreasing in MPTP mice as the typical symptom of Parkinson's disease. A mouse's nerve cells containing TH for generation of dopamine could be damaged due to Parkinson's disease. The test results suggest that menthol proves effective in protecting nerve cells from decrease in the level of dopamine.

In the Parkinson's disease test of Embodiment 1, exercise performance of mice worsened by Parkinson's disease is improved by topical menthol as the therapy of a neurodegenerative disease that recovers the level of dopamine in a mouse's brain and protects nerve cells.

### Embodiment 2: topical menthol for improvement of a stoke

### 1. Experimental design and creation of an ischemic stroke mouse model

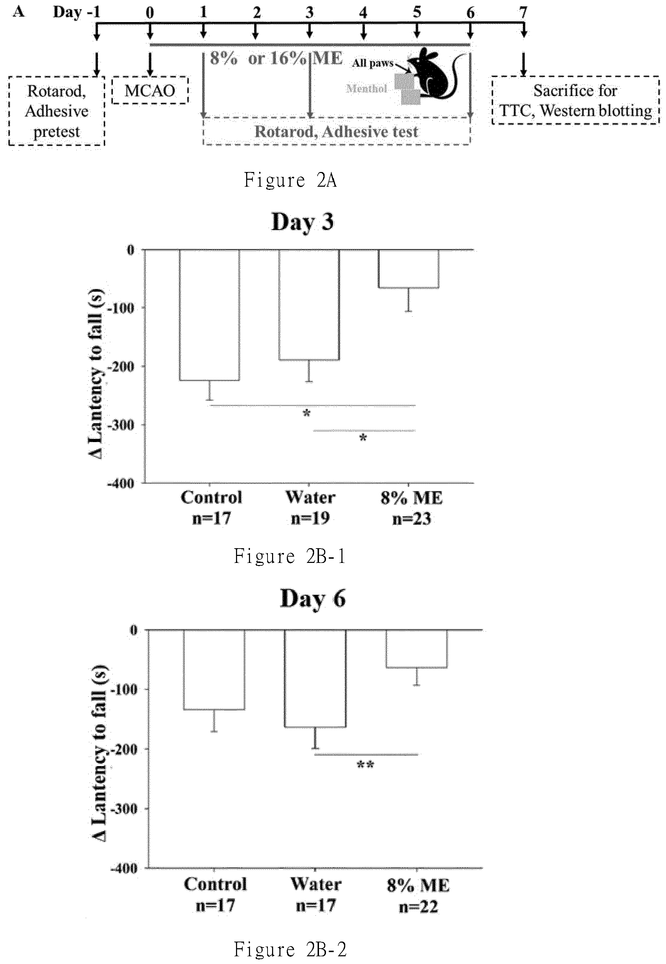

Laboratory mice are divided into four groups: "Control" consisting of mice processed through MCAO for the ischemic stroke; "Water" consisting of mice which are processed for the ischemic stroke through MCAO and whose limbs are soaked in water; "MCAO+8%ME" consisting of mice which are processed for the ischemic stroke through MCAO and whose limbs are soaked in 8% menthol; "MCAO+16%ME" consisting of mice which are processed for the ischemic stroke through MCAO and whose limbs are soaked in 16% menthol. In Embodiment 2, menthol is prepared with 95% ethanol. Referring to the experiment schedule in FIG. 2A, mice are sacrificed on Day 7 for following analyses. Mice are processed for the ischemic stroke through MCAO on Day 0 and administrated with menthol once per day from Day 0 to Day 6. On the day of processing mice through MCAO, the limbs of a mouse resting 20 minutes after the operation are soaked in menthol for one minute; a mouse's limbs are soaked in menthol for 15 seconds only from Day 2 to Day 6; comparatively, a Water mouse's limbs are soaked in water. The sensorimotor nerve function test is conducted for mice one day before MCAO processing (i.e., pre-MCAO) and 1, 3 and 6 days after MCAO processing; the cerebral infarction volume is measured for checking any improvement in the stroke due to menthol on Day 7 after MCAO processing.

### 2. Sensorimotor nerve function test

### 2.1 Rotarod test

In the rotarod test, a mouse is placed on a self-accelerated rotating rod (rotation rate from 4 to 40rpm) on Day 3 and Day 6 for measuring the retention time (FIG. 2B-1 and FIG. 2B-2) and comparing test results with those of "pre-MCAO". Mice in each group are tested three times. The test results are analyzed with the one-way ANOVA for further reviews through the multiple comparison analysis (Dunnett's method) and statistics of between-group variances (* p<0.05). The asterisk (*) means a significant difference between "Control" and "Water" or "MCAO+8%ME" (* p<0.05; ** p<0.01). As shown in test results, the symptom of sensorimotor disorder attributed to the ischemic stroke is recognized because the retention time performed by MCAO mice on Day 3 (Day 6) decreases significantly. Comparatively, the retention time performed by mice processed with menthol longer than the retention time performed by MCAO or Water mice suggests the sensorimotor neuron function of paralytic mice is improved.

### 2.2 Adhesive removal test

The adhesive removal test is conducted on Day 1, 3 and 6 and the test results are compared with status at "pre-MCAO". As shown in the experimental process, the time of a mouse finding and removing 4 (mm)×4 (mm) stickers, which have been adhered to its forepaws outside a test cage, is recorded (FIG. 2C-1, FIG. 2C-2) for statistic analysis as described in Section 2.1 (* p<0.05; ** p<0.01; *** p<0.001). It can be seen from statistic analysis between groups that time spent in removing stickers by mice to which menthol is administered is significantly shorter than time spent by Control or Water mice and significantly less time spent in finding stickers by mice to which menthol is administered than Water mice on Day 6 suggests better reaction speed and correction of stroke-induced sensorimotor disorder after administration of menthol.

### 3. Measurement of the cerebral infarction volume

After a mouse in each group is sacrificed through perfusion one week after MCAO processing, a specimen of the mouse's living brain is stained by TTC (FIG. 2D-2) for measurement of the cerebral infarction volume (mm3) (FIG. 2D-1). The test results for the cerebral infarction volume are analyzed with the one-way ANOVA for further reviews through the multiple comparison analysis (Tukey's method) and statistics of between-group variances (* p<0.05). As shown in test results, the cerebral infarction volume of mice administered with menthol significantly decreases in contrast to other mice in Control and Water groups.

It is demonstrated in **Embodiment 1** for the Parkinson's disease mouse model that the impaired sports coordination due to Parkinson's disease and the level of dopamine in a mouse's brain are improved with topical menthol administrated to laboratory mice. Moreover, topical menthol with the effect of neuroprotection prevents dopamine neurons in substantia nigra from impairments, raising the level of TH. As shown in **Embodiment 2** for the ischemic stroke mouse model, the cerebral infarction volume decreases within seven days for correction of the defect in sensorimotor nerves with topical menthol administrated to laboratory mice.

The present application discloses menthol applied topically for improvement of Parkinson's disease and strokes. As indicated in the present disclosure, menthol which proves effective in raising the level of dopamine in a mouse's brain, protecting nerves and subduing the cerebral infarction volume is applicable to improving other neurodegenerative diseases, for example, Alzheimer's disease. Any isomer of menthol or a compound with a similar chemical structure, each of which has approximate functionality known to a person with common knowledge in the art, should be incorporated into claims hereinafter.

As shown in embodiments disclosed herein, a topical composition is prepared with menthol for moderations of neurodegenerative diseases, stroke or other symptoms attributed to cerebral neurons impaired or degenerated, shortage of dopamine or stroke and including, without limitation, cognitive dysfunction, ataxia, depression, weakness of limbs, hemiplegia, headache, vertigo, aphasia or alalia, blurring of vision or visual impairment, palpitation, convulsion, nausea and vomiting, fatigue, drooling, dysphagia, facial paralysis and incontinence. In these embodiments of the present disclosure, the effective percentage of menthol in the optical composition to be applied on a human body ranges from 1% to 20% according to a dose conversion between different animal models.

The topical composition, which comprises an adjuvant, a diluent, a carrier, each of which is accepted pharmaceutically, and other added ingredients effective in cosmetics or health preserving due to the cooling effect of menthol, is applicable to improvement, cool sense, health care and cosmetics and manufactured as patches, liquids, pastes, oily substances, powders, gels, sprays, composite products or other products to be covered on limbs and applied on skin. A product to be covered on limbs can be a glove (FIG. 3A), a foot muff (FIG. 3B), a sock or an extended part or a layered object from a garment for continuous contact between menthol and skin. The objects in FIGS. 3A and 3B are probable examples embodied in the patent application and any transformation from or change or modification in materials or exteriors of an object without departing from the above concept should be incorporated in claims hereinafter.

### 4. Difference in efficiency between topical and oval products

Laboratory mice are divided into five groups: "Water" consisting of mice which are processed for the ischemic stroke through MCAO and whose limbs are soaked in water; "8%ME" consisting of mice which are processed for the ischemic stroke through MCAO and whose limbs are soaked in 8% menthol; "16%ME" consisting of mice which are processed for the ischemic stroke through MCAO and whose limbs are soaked in 16% menthol; "Back-ME" consisting of mice which are processed for the ischemic stroke through MCAO and whose backs are soaked in menthol; "Oral-ME" consisting of mice which are processed for the ischemic stroke through MCAO and fed with menthol. Menthol is prepared with 95% ethanol; the experimental design and data analyses are disclosed in the patent application; the experimental schedule is shown in FIG. 4A. These mice are sacrificed on Day 7 for following analyses. As shown in FIGS. 4B-1 and 4B-2, the test results are analyzed with the one-way ANOVA for further reviews through the multiple comparison analysis (Duncan's method) and statistics of between-group variances (* p<0.05; *: comparison with the "Water" group; †: comparison with the "16%ME" group; ‡: comparison with the "8%ME" group). In the rotarod test, the retention time performed by mice which are fed with oral menthol or whose backs are administered with topical menthol is not extended effectively. Comparatively, the fact that the retention time performed by mice whose limbs are soaked in menthol is longer than the retention time performed by Back-ME, Water or Oral-ME mice suggests the sensorimotor neuron function of paralytic mice is improved by topical menthol applied on mice's limbs. As shown in FIGS. 4C-1 and 4C-2 for the adhesive removal test, the fact that time spent in finding and removing stickers by mice whose limbs are soaked in menthol is significantly shorter than other mice suggests much better reaction speed of paralytic mice whose sensorimotor defects induced by a stroke are corrected by topical menthol compared with other mice which are fed with oral menthol or whose backs are soaked in menthol. As shown in FIGS. 4D-1 and 4D-2 for measurement of the cerebral infarction volume, the cerebral infarction volume measured in mice whose limbs are soaked in menthol is significantly subdued in contrast to the unchanged cerebral infarction volume of other mice which are fed with oral menthol, whose backs are administered with menthol or whose limbs are soaked in water. Accordingly, topical menthol applied on paralytic mice's limbs as shown in the patent application contributes to improving the sensorimotor neuron function, correcting sensorimotor defects of paralytic mice and subduing the cerebral infarction volume but these phenomena for the ischemic stroke improved are unavailable in mice which are fed with oral menthol or whose backs are administered with topical menthol. That is, other topical medications administered through specific approaches for the same therapeutic effect as disclosed in the patent application should be studied for these mice whose symptoms are not improved significantly.

In embodiments of the present disclosure, a topical composition is provided to improve diseases or symptoms attributed to cerebral neurons impaired or degenerated, shortage of dopamine in a brain or stroke. Any idea based on administration of topical menthol or an isomer thereof to improve diseases or symptoms without departing from the present disclosure should be regarded as an equivalent embodiment and incorporated into claims hereinafter.

The embodiments disclosed hereinbefore are aimed to describe technical ideas and features in the present disclosure and make persons skilled in the art comprehend and embody the present invention but not taken as evidences to restrict claims hereinafter, that is, any equivalent change or modification depending on spirit of the present disclosure are still incorporated into the claims.

## Claims

1. Use of menthol or an isomer thereof for manufacture of a topical composition for improving neurodegenerative diseases and stroke.

2. The use according to claim 1, wherein the isomer of menthol is a structural isomer or a stereoisomer.

3. The use according to claim 1, wherein the menthol or the isomer thereof in the topical composition features a weight percentage ranging from 1% to 20%.

4. The use according to claim 1, wherein the topical composition is administrated once or twice per day.

5. The use according to claim 1, wherein the topical composition is manufactured as patches, liquids, pastes, oily substances, powders, gels, sprays, composite products or other products to be covered on limbs and applied on skin.

6. The use according to claim 5, wherein a product to be covered on limbs can be a glove, a foot muff, a sock or an extended part or a layered object from a garment.

7. The use according to claim 5, wherein a product to be covered on limbs is manufactured for continuous contact between skin and menthol.

8. The use according to claim 1, wherein the neurodegenerative diseases are attributed to cerebral neurons impaired or degenerated or shortage of dopamine in a brain.

9. The use according to claim 1 or 8, wherein one of the neurodegenerative diseases is Parkinson's disease or Alzheimer's disease and the stroke is ischemic stroke.

10. The use according to claim 1, wherein the topical composition is used to improve symptoms attributed to cerebral neurons impaired or degenerated, shortage of dopamine or stroke and including, without limitation, cognitive dysfunction, ataxia, depression, weakness of limbs, hemiplegia, headache, vertigo, aphasia or alalia, blurring of vision or visual impairment, palpitation, convulsion, nausea and vomiting, fatigue, drooling, dysphagia, facial paralysis and incontinence.

11. Use of menthol or an isomer thereof for manufacture of a topical composition for improving diseases or symptoms attributed to cerebral neurons impaired or degenerated.

12. Use of menthol or an isomer thereof for manufacture of a topical composition for improving diseases or symptoms attributed to shortage of dopamine in a brain.

13. Use of menthol or an isomer thereof for manufacture of a topical composition for improving diseases or symptoms attributed to stroke.
